# FASCICULE DE BREVET EUROPEEN

(11) **EP 3 589 622 B1**
(45) Date de publication et mention de la délivrance du brevet: **31.03.2021**
(21) Numéro de dépôt: 18712446.6
(22) Date de dépôt: 16.02.2018
(51) Int. Cl.: C07D 279/18

(54) **PROCEDE DE PREPARATION DE L'IODURE DE 3,7-BIS(DIMETHYLAMINO)PHENOTHIAZINE-5-YLIUM**
VERFAHREN ZUR HERSTELLUNG VON 3,7-BIS(DIMETHYLAMINO)PHENOTHIAZIN-5-YLIUM-IODID
PROCESS FOR THE PREPARATION OF 3,7-BIS(DIMETHYLAMINO)PHENOTHIAZINE-5-YLIUM IODIDE

(30) Priorité: 02.03.2017 FR 1751700
(43) Date de publication de la demande: 08.01.2020
(73) Titulaire: Provepharm Life Solutions, 13013 Marseille (FR)
(72) Inventeur: FERAUD, Michel, 13013 Marseille (FR); SAYAH, Babak, 13013 Marseille (FR); QUERU, Stéphane, 13013 MArseille (FR); LAURENT, Marina, 13013 Marseille (FR)
(74) Mandataire: Corizzi, Valérie
(86) Numéro de dépôt international: PCT/FR2018/050372
(87) Numéro de publication internationale: WO 2018/158520

(56) Documents cités:
- WO-A1-2005/054217
- ANITA GOLLMER ET AL: "A novel set of symmetric methylene blue derivatives exhibits effective bacteria photokilling - a structure-response study", PHOTOCHEMICAL AND PHOTOBIOLOGICAL SCIENCES, vol. 14, no. 2, 1 janvier 2015 (2015-01-01), pages 335-351, XP055368267, GB ISSN: 1474-905X, DOI: 10.1039/C4PP00309H cité dans la demande
- MELLISH K J ET AL: "In vitro photodynamic activity of a series of methylene blue analogues", 20020101, vol. 75, no. 4, 1 janvier 2002 (2002-01-01), pages 392-397, XP002990046, cité dans la demande

## Description

La présente invention concerne un nouveau procédé de préparation de l'iodure de 3,7-bis(diméthylamino)phénothiazine-5-ylium, ce procédé permettant d'obtenir un produit présentant un degré de pureté élevé, tout en étant très simple de mise en œuvre et présentant des rendements élevés. Ce procédé permet d'avoir accès à d'autres halogénures, notamment au chlorure de 3,7-bis(diméthylamino)phénothiazine-5-ylium, en quelques étapes. Ainsi ce procédé constitue la première étape d'une voie d'accès à un bleu de méthylène de qualité élevée et avec de bons rendements.

### Etat de la technique antérieure

On connaît de l'art antérieur différents procédés de synthèse de dérivés de phénothiazine, substitués en position 3 et en position 7, par des groupements identiques ou non. Tous ces procédés ont en commun d'être réalisés en plusieurs étapes.

Le document « A synthetic Route to 3-(Dialkylamino)phenothiazin-5-ium Salts and 3,7-Disubstituted Derivatives Containing Two Différent Amino Groups » (L. Strekowski, D. F. Hou and R. L. Wydra ; Journal of Heterocyclic Chemistry ; 1993 ; 30 ; 1693-1695) décrit la préparation en plusieurs étapes de dérivés de phénothiazine, substitués en positions 3 et 7 par des groupements distincts. La première étape de ce procédé consiste à transformer la phénothiazine en périodure, également connu sous le nom de tétraiodure de phénothiazine-5-ylium, qui est purifié et isolé. La seconde étape de ce procédé consiste à ajouter deux équivalents molaires de dialkylamine au périodure, afin d'obtenir un dérivé de phénothiazine substitué en position 3 par une dialkylamine. Un traitement par au moins quatre équivalents molaires d'une autre dialkylamine permet d'obtenir un dérivé asymétrique de phénothiazine substitué en positions 3 et 7. Ce procédé a été adapté à la synthèse d'iodures de 3,7-bis(dialkylamino)phénothiazine-5-ylium par K.J. Mellish et al., Photochemistry and Photobiology, 2002, 75(4) ; 392-397, avec des alkyles en C2 à C6. Ce procédé fait appel à l'utilisation de solvants tels que chloroforme dont l'utilisation à l'échelle industrielle est peu souhaitable. En outre, les rendements sont au mieux de 55 %.

Le document « A novel set of symmetric methylene blue derivative exhibits effective bacteria photokilling - a structure - response study » (Anita Gollmer, et al., Photochem. Photobiol. Sci. ; vol.14, n°2, 1 janvier 2015, p.335-351) est le seul à décrire la préparation multi-étapes du chlorure de 3,7-bis(diméthylamino)phénothiazine-5-ylium via le périodure de 3,7-bis(diméthylamino)phénothiazine-5-ylium. La première étape de ce procédé consiste à transformer la phénothiazine en périodure qui est purifié et isolé. La seconde étape consiste à traiter le periodure par la diméthylamine dissoute dans un mélange de méthanol et de dichlorométhane. Un échange d'ion permet d'obtenir le chlorure de 3,7-bis(diméthylamino)phénothiazine-5-ylium. Aucun mode opératoire précis et reproductible n'est divulgué. Ce procédé fait appel à l'utilisation de solvants tels que le dichlorométhane dont l'utilisation à l'échelle industrielle est peu souhaitable. Lorsque l'on a tenté de reproduire ce procédé, on a obtenu des rendements faibles (42,7%) et un produit présentant une pureté de 85,36% (CLHP).

Le document N. Leventis et al., Tetrahedron 1997 vol. 53, N° 29, 10083-10092, 1997 décrit une synthèse du bromure de 3,7-bis(diméthylamino)phénothiazine-5-ylium en deux étapes : en premier lieu un traitement par un large excès de brome dans de l'acide acétique permet de former le bromure de 3,7-bis(dibromo)phénothiazine-5-ylium, suivi d'un traitement par de la diméthylamine. La première étape de ce procédé présente de nombreux inconvénients : l'utilisation d'un large excès (20 équivalents) de brome et d'acide acétique débarrassé d'oxygène, ainsi que le caractère instantané de la réaction de bromation, difficilement contrôlable, sont peu compatibles avec une application à l'échelle industrielle. La seconde étape de ce procédé fait appel à l'utilisation de solvants tels que chloroforme dont l'utilisation à l'échelle industrielle est peu souhaitable. Le produit doit être purifié par chromatographie sur colonne de silice, méthode peu adaptée à la production de quantités importantes de produit.

Le chlorure de 3,7-bis(diméthylamino)phénothiazine-5-ylium est un composé utilisé de longue date comme colorant et indicateur redox, comme révélateur optique dans les systèmes biophysiques, dans des matériaux nanoporeux comme matériau séparateur, et en imagerie photoélectrochimique. Il est également connu pour ses applications comme agent antiseptique, antiinfectieux, comme antidote et comme agent de diagnostique. Il trouve des utilisations notamment en gynécologie, néonatologie, cancérologie, oncologie, urologie, ophtalmologie et gastro-entérologie, la réduction des contaminants pathogéniques dans le sang (GB 2373787). De nouvelles utilisations dans le domaine thérapeutique sont en cours de développement, telle que la prévention ou l'inhibition d'une réaction hémodynamique excessive (WO 03/082296), le traitement de la maladie d'Alzheimer et plus généralement le traitement des maladies dégénératives du système nerveux central (WO 2008/007074).

Pour ces applications, il est nécessaire de disposer d'une composition de bleu de méthylène comprenant peu d'impuretés organiques et métalliques.

Plusieurs procédés connus de préparation du bleu de méthylène font appel à l'utilisation de réactifs métalliques (WO 2005/054217; WO 2006/032879) et conduisent à un produit contaminé par des résidus métalliques en quantité importante. La réduction du taux de ces impuretés requiert des étapes de purification fastidieuses.

Elle est limitée par le caractère complexant des métaux de la molécule de 3,7-bis(diméthylamino)phénothiazine-5-ylium.

Le bleu de méthylène et ses impuretés organiques : Azure A, Azure B et Azure C, présentent des structures très proches qui rendent leur séparation difficile par des techniques classiques de séparation.

Des méthodes de purification du bleu de méthylène, afin d'en éliminer les contaminants métalliques et organiques, ont été décrites (WO 2008/007074 ; WO 2008/006979). Toutefois, la synthèse du bleu de méthylène brut, auquel on applique ces méthodes de purification, passe par l'utilisation de réactifs toxiques comme des dérivés du chrome.

Par conséquent, il subsiste donc le besoin d'un procédé permettant d'accéder directement à un halogénure de 3,7-bis(diméthylamino)phénothiazine-5-ylium de pureté élevée, avec des rendements satisfaisants, ce procédé ne faisant pas appel à des réactifs hautement toxiques tels que les oxydes de chrome

Il existe, en particulier le besoin d'un procédé permettant de préparer du bleu de méthylène pour une utilisation dans le domaine thérapeutique grâce à un procédé simple de mise en œuvre, avec un rendement et un degré de pureté élevés.

L'iodure de 3,7-bis(diméthylamino)phénothiazine-5-ylium permet d'accéder facilement, par différentes méthodes de transformation, au chlorure de 3,7-bis(diméthylamino)phénothiazine-5-ylium.

L'objectif de l'invention a été de mettre au point un nouveau procédé de préparation de l'iodure de 3,7-bis(diméthylamino)phénothiazine-5-ylium. Un tel procédé permet d'obtenir du chlorure de 3,7-bis(diméthylamino)phénothiazine-5-ylium ou bleu de méthylène, par simple échange d'ions ou par d'autres méthodes connues qui seront décrites ci-dessous.

Le demandeur a cherché à mettre au point un procédé de synthèse de l'iodure de 3,7-bis(diméthylamino)phénothiazine-5-ylium qui soit rapide, peu coûteux, efficace, avec un rendement et un degré de pureté élevé et facilement extrapolable à l'échelle industrielle.

### Résumé de l'invention

L'invention concerne un procédé de préparation d'iodure de 3,7-bis(diméthylamino)phénothiazine-5-ylium, ce procédé utilisant la phénothiazine comme produit de départ et comprenant les étapes suivantes :
a) Le traitement de la phénothiazine par du di-iode,
b) Le traitement du milieu réactionnel directement issu de l'étape a) par de la diméthylamine.

Selon un mode de réalisation préféré, le traitement par le di-iode est mis en œuvre avec une quantité de di-iode par rapport à la phénothiazine allant de 2,5 équivalents molaires à 3,5 équivalents molaires.

Encore plus avantageusement, le traitement par le di-iode est mis en œuvre avec une quantité de di-iode par rapport à la phénothiazine allant de 2,9 équivalents molaires à 3,3 équivalents molaires.

Selon un mode de réalisation préféré, avant l'étape b), le milieu réactionnel issu de l'étape a) est conditionné à une température allant de 5°C à 50°C, de préférence de 10°C à 45°C, encore mieux de 20°C à 35°C.

Selon un mode de réalisation préféré, le traitement par de la diméthylamine est mis en œuvre avec au moins 7 équivalents molaires de diméthylamine par rapport à la phénothiazine.

Selon un mode de réalisation préféré, à l'étape a), le solvant est choisi parmi : un solvant aromatique ou l'acétonitrile, ou leurs mélanges, de préférence le toluène ou l'acétonitrile, ou leurs mélanges.

Selon un mode de réalisation préféré, à l'étape b), la diméthylamine est introduite dans le milieu réactionnel sous forme de solution dans de l'eau.

Selon un mode de réalisation préféré, il se forme un précipité à l'issue du traitement de l'étape b), ledit précipité étant récupéré par filtration.

L'invention concerne encore l'utilisation du procédé décrit ci-dessus, et de façon détaillée ci-dessous, pour produire une composition comprenant de l'iodure de 3,7-bis(dialkylamino)phénothiazine-5-ylium, dans laquelle l'iodure de 3,7-bis(dialkylamino)phénothiazine-5-ylium représente au moins 95% de la composition, le % étant mesuré par CLHP avec une détection à 246 nm.

L'invention concerne également un procédé de fabrication du chlorure de 3,7-bis(diméthylamino)phénothiazine-5-ylium, ce procédé comprenant :
i) La production d'iodure de 3,7-bis(diméthylamino)phénothiazine-5-ylium suivant le procédé décrit ci-dessus, et de façon détaillée ci-dessous,
ii) La transformation de l'iodure de 3,7-bis(diméthylamino)phénothiazine-5-ylium en chlorure de 3,7-bis(diméthylamino)phénothiazine-5-ylium.

L'invention concerne encore un procédé de fabrication d'un médicament comprenant du chlorure de 3,7-bis(diméthylamino)phénothiazine-5-ylium, ce procédé comprenant la production du chlorure de 3,7-bis(diméthylamino)phénothiazine-5-ylium suivant le procédé décrit ci-dessus, et de façon détaillée ci-dessous, et son introduction dans un milieu pharmaceutiquement acceptable.

Selon un mode de réalisation préféré, le procédé de fabrication d'un médicament concerne la fabrication d'un médicament destiné à la prévention ou au traitement d'une pathologie sélectionnée parmi: une tauopathie, une maladie d'agrégation de protéine tau, la maladie de Pick, la paralysie supranucléaire progressive (PSP), la démence fronto-temporale (FTD), la FTD et le parkinsonisme lié au chromosome 17 (FTDP-17), le complexe de désinhibition-démence-parkinsonisme-amyotrophie (DDPAC), la dégénérescence pallido-ponto-nigrale (PPND), le syndrome de Guam-ALS, la dégénérescence pallido-nigro-luysian (PNLD), la dégénérescence cortico-basale (CBD), une déficience cognitive légère (MCI), le cancer de la peau, le mélanome, la méthémoglobinémie, une infection virale, une infection bactérienne, une infection à protozoaires, une infection parasitaire, le paludisme, la leishmaniose viscérale, la maladie africaine du sommeil, la toxoplasmose, la giardiase, la maladie de Chagas, une infection par le virus de l'hépatite C (VHC), une infection par le virus de l'immunodéficience humaine (VIH), une infection par le virus du Nil occidental (WNV), une synucléinopathie, la maladie de Parkinson (PD), la démence à corps de Lewy (DLB), une atrophie multisystématisée (MSA), une parkinsonisme d'origine médicamenteuse, une dysautomie pure (PAF), un choc septique, une réaction hémodynamique excessive, le cancer du sein, les troubles maniaco-dépressifs, la maladie d'Alzheimer (AD) et plus généralement le traitement des maladies dégénératives du système nerveux central.

L'expression « consiste essentiellement en » suivie d'une ou plusieurs caractéristiques, signifie que peuvent être inclus dans le procédé ou le matériau de l'invention, outre les composants ou étapes explicitement énumérés, des composants ou des étapes qui ne modifient pas significativement les propriétés et caractéristiques de l'invention.

### Description détaillée

L'invention concerne un procédé de transformation de la phénothiazine en iodure de 3,7-bis(diméthylamino)phénothiazine-5-ylium en deux étapes mises en œuvre dans un même milieu réactionnel.

Ce procédé de transformation comprend :
a) le traitement de la phénothiazine par du di-iode ;
b) le traitement du milieu réactionnel directement issu de l'étape a) par de la dialkylamine.

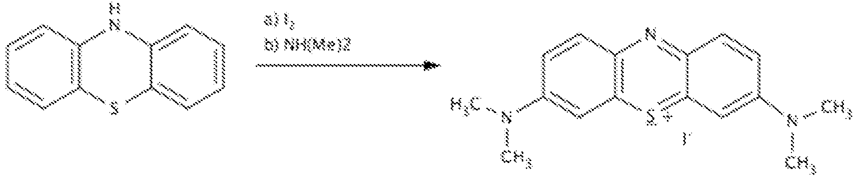

Ce procédé de préparation permet d'obtenir de l'iodure de 3,7-bis(diméthylamino)phénothiazine-5-ylium, avec un degré de pureté élevé, tout en étant très simple de mise en œuvre et présentant des rendements élevés.

Il est connu de l'art antérieur que l'ajout de di-iode à une phénothiazine conduit à une réaction d'oxydation.

L'oxydation de la phénothiazine par l'iode permet de former du périodure, produit intermédiaire de la réaction, qui, dans les procédés antérieurs, est purifié et isolé.

L'invention concerne un procédé de préparation d'iodure de 3,7-bis(diméthylamino)phénothiazine-5-ylium qui ne nécessite pas d'isoler et de purifier le périodure.

En effet, on a constaté que l'isolement et la purification du périodure dans une étape supplémentaire conduit, après traitement par de la diméthylamine, à un iodure de 3,7-bis(diméthylamino)phénothiazine-5-ylium de qualité peu satisfaisante et qu'un tel procédé présente des rendements faibles.

Le procédé de l'invention se caractérise en ce que les deux étapes essentielles sont mises en œuvre dans le même milieu réactionnel. Un tel procédé est également désigné habituellement de « one-pot », littéralement « un réacteur », ce qui signifie que la succession de réactions est mise en œuvre sans isolement ou purification, complet ou partiel, du produit de l'étape a) avant l'engagement de l'étape b). La composition du milieu réactionnel évolue au cours du temps, mais les produits intermédiaires ne sont pas isolés et/ou purifiés, seul le produit final est séparé du milieu réactionnel. Dans le procédé de l'invention, au moins deux réactions successives se produisent, la composition du milieu réactionnel évolue au cours du temps, mais le periodure de phénothiazine n'est pas isolés et/ou purifié, seul l'iodure de 3,7-bis(diméthylamino)phénothiazine-5-ylium est séparé du milieu réactionnel.

Un tel procédé présente l'avantage d'être facile à mettre en œuvre et de nécessiter peu de manipulations. Habituellement, les procédés one-pot ont la réputation de conduire à des mélanges moins purs que ceux procédant par isolement et purification des intermédiaires. Toutefois, dans le cas présent, de façon surprenante, on a constaté que le procédé en deux étapes a) et b) mis en œuvre dans un même milieu réactionnel conduit à un produit de pureté plus élevée qu'un procédé similaire réalisé avec isolement et purification du periodure de phénothiazine.

### La phénothiazine :

Le produit de départ est la phénothiazine qui est un produit commercial.

De préférence, la phénothiazine utilisée comme produit de départ présente une pureté organique supérieure ou égale à 98% (en % d'aire) mesurée par chromatographie liquide à haute performance, avec une détection à 246 nm.

Avantageusement, on utilise une phénothiazine comportant peu ou pas d'impuretés métalliques. De préférence, on utilise une phénothiazine comportant moins de 200 ppm de contaminants métalliques, avantageusement moins de 100 ppm de contaminants métalliques, encore mieux, moins de 50 ppm de contaminants métalliques, et encore plus avantageusement moins de 20 ppm de contaminants métalliques.

Par contaminants métalliques on entend tous les métaux de la classification périodique des éléments et notamment : Cd, Cr, Hg, Mn, Ni, Sn, Pb, Al, Fe, Cu, Zn, As, Mo, Mg, Ti, V, U, Co. Plus particulièrement, on entend par contaminants métalliques les métaux dits « lourds » et en particulier : Al, Cd, Cr, Cu, Sn, Mn, Hg, Mo, Ni, Pb, Zn.

### Etape a) : Traitement de la phénothiazine par du di-iode :

Selon l'invention, les étapes a) et b) sont mises en œuvre dans un solvant ou dans un mélange de solvants.

A l'étape a), on peut utiliser un seul solvant ou un mélange de solvants. A l'étape b), la diméthylamine est introduite dans le milieu réactionnel sous forme de solution dans un solvant qui peut être identique ou différent de celui de l'étape a).

La phénothiazine est traitée par du di-iode, puis par de la diméthylamine, dans un solvant, ou dans un mélange de solvants, qui est choisi notamment pour sa capacité à solubiliser la phénothiazine et le di-iode.

Parmi les solvants utilisables dans le procédé de l'invention, on peut citer : les alcools, comme le méthanol, l'éthanol ; le tétrahydrofurane ; les solvants aromatiques, comme le toluène, le xylène, et l'éthylbenzène ; l'acétonitrile ; les mélanges de ces solvants.

De préférence, le traitement de la phénothiazine par le di-iode est mis en œuvre dans un solvant choisi parmi les solvants aromatiques et l'acétonitrile.

Avantageusement, le solvant est choisi parmi le toluène et l'acétonitrile.

Avantageusement, le traitement de la phénothiazine par le di-iode est mis en œuvre avec au moins 2,5 équivalents molaires et au plus 3,5 équivalents molaires de di-iode par rapport à la phénothiazine. En dehors de cette gamme de valeurs, on a constaté une baisse significative des rendements en iodure de 3,7-bis(diméthylamino)phénothiazine-5-ylium. De préférence, le traitement de la phénothiazine par le di-iode est mis en œuvre avec au moins 2,7 équivalents molaires et au plus 3,3 équivalents molaires de di-iode par rapport à la phénothiazine, encore mieux avec au moins 2,8 équivalents molaires et au plus 3,2 équivalents molaires de di-iode par rapport à la phénothiazine.

De préférence, le traitement de la phénothiazine par le di-iode est mis en œuvre avec environ trois équivalents molaires de di-iode par rapport à la phénothiazine.

Selon un premier mode de réalisation préféré l'étape a) est mise en œuvre dans du toluène en présence d'au moins 2,5 équivalents molaires et au plus 3,5 équivalents molaires de di-iode par rapport à la phénothiazine.

De préférence, l'étape a) est mise en œuvre dans du toluène en présence d'au moins 2,7 équivalents molaires et au plus 3,3 équivalents molaires de di-iode par rapport à la phénothiazine, encore mieux avec au moins 2,8 équivalents molaires et au plus 3,2 équivalents molaires de di-iode par rapport à la phénothiazine.

Selon un second mode de réalisation préféré l'étape a) est mise en œuvre dans de l'acétonitrile en présence d'au moins 2,5 équivalents molaires et au plus 3,5 équivalents molaires de di-iode par rapport à la phénothiazine.

De préférence, l'étape a) est mise en œuvre dans de l'acétonitrile en présence d'au moins 2,7 équivalents molaires et au plus 3,3 équivalents molaires de di-iode par rapport à la phénothiazine, encore mieux avec au moins 2,8 équivalents molaires et au plus 3,2 équivalents molaires de di-iode par rapport à la phénothiazine.

De préférence, l'étape a) est réalisée sous agitation, à une température allant de la température ambiante à 100°C. Cette étape n'étant pas exothermique, les réactifs et le ou les solvants sont introduits dans le réacteur qui est ensuite conditionné à la température choisie.

Avantageusement, le traitement de la phénothiazine par le di-iode est mis en œuvre à une température allant de 30°C à 90°C, avantageusement de 40°C à 80°C, encore mieux de 50°C à 70°C.

De préférence, la durée totale de traitement de la phénothiazine par le di-iode est de 15 minutes à 6h, avantageusement de 30 minutes à 4h, encore mieux de 1 h à 3h.

Il est possible de suivre l'évolution de la réaction par chromatographie sur couche mince ou par CLHP (chromatographie liquide à haute performance).

Avantageusement, le traitement de la phénothiazine par le di-iode est mis en œuvre :
- à une température allant de 30°C à 90°C pendant une durée de 15 minutes à 6h,
- préférentiellement à une température allant de 30°C à 90°C pendant une durée de 30 minutes à 4h,
- avantageusement à une température de 40°C à 80°C pendant une durée de 1h à 3h.

A la différence de l'art antérieur, le milieu réactionnel obtenu à l'issue de cette réaction est directement soumis à un traitement par de la diméthylamine, sans isoler et/ou purifier de façon partielle ou totale le produit intermédiaire.

### Etape b) Traitement du milieu réactionnel par de la diméthylamine

On ajoute au milieu réactionnel issu de l'étape a), de la diméthylamine NH(CH₃)₂, qui est avantageusement introduite sous forme de solution dans un solvant.

La solution de diméthylamine peut être dans un solvant ou un mélange de solvants choisis parmi : l'eau, le tétrahydrofurane, le méthanol, l'éthanol, un mélange de ces solvants.

De préférence, la diméthylamine est introduite dans le milieu réactionnel sous forme de solution dans de l'eau ou dans du tétrahydrofurane ou dans un mélange de ces solvants.

Encore plus préférentiellement, la diméthylamine est introduite dans le milieu réactionnel sous forme de solution dans l'eau.

Selon un premier mode de réalisation préféré, le milieu réactionnel après l'introduction de la diméthylamine comprend un mélange de toluène et d'eau dans un ratio volumique allant de 99/1 à 50/50, avantageusement de 95/5 à 60/40, encore mieux de 90/10 à 70/30.

Selon un second mode de réalisation préféré, le milieu réactionnel après l'introduction de la diméthylamine comprend un mélange de d'acétonitrile et d'eau dans un ratio volumique allant de 99/1 à 50/50, avantageusement de 95/5 à 60/40, encore mieux de 90/10 à 70/30.

De préférence, le traitement par la diméthylamine est mis en oeuvre avec au moins six équivalents molaires de diméthylamine par rapport à la phénothiazine, avantageusement au moins sept équivalents molaires de diméthylamine par rapport à la phénothiazine, encore plus avantageusement au moins huit équivalents molaires de diméthylamine par rapport à la phénothiazine.

De préférence, le traitement par la diméthylamine est mis en oeuvre avec de six à quinze équivalents molaires de diméthylamine par rapport à la phénothiazine, avantageusement de sept à douze équivalents molaires de diméthylamine par rapport à la phénothiazine, encore plus avantageusement de huit à douze équivalents molaires de diméthylamine par rapport à la phénothiazine.

L'ajout de diméthylamine conduit à une réaction exothermique.

Avantageusement, la température du milieu réactionnel est contrôlée avant l'introduction de la diméthylamine. De préférence, la température du milieu réactionnel est de 1°C à 50°C au moment de l'introduction de la diméthylamine, préférentiellement elle est de 2°C à 40°C, encore mieux de 5°C à 30°C.

L'exothermie de la réaction conduit à une élévation de la température du milieu réactionnel pendant la durée d'introduction de la diméthylamine. Après que toute la diméthylamine a été versée dans le milieu réactionnel, la température se stabilise. Avantageusement, la température du milieu réactionnel est alors contrôlée et maintenue à 20-25°C.

De préférence, le milieu réactionnel est ensuite maintenu sous agitation et à une température allant de 20 à 25°C pendant 20 minutes à 6h, de préférence de 1h à 5h, encore mieux de 2h à 4h.

L'ajout de diméthylamine dans le milieu réactionnel conduit à un changement de coloration de ce milieu, qui passe du marron au bleu foncé.

Selon l'invention, on observe au bout de ce temps la formation d'un solide dans le milieu réactionnel. Il est possible de suivre l'évolution de la réaction par CLHP.

Après la réaction avec la diméthylamine, il est possible, pour favoriser la précipitation de l'iodure de 3,7-(dialkylamino)phénothiazine-5-ylium, de traiter le milieu réactionnel par ajout d'un autre solvant dans lequel il n'est pas ou peu soluble. En fonction du choix du solvant dans lequel a été fait la réaction, on peut utiliser au choix : du toluène, du tétrahydrofurane, de l'acétonitrile, de l'éthanol, de l'acétone, de l'eau ou un mélange des ces solvants.

Selon un premier mode de réalisation préféré, le milieu réactionnel comprend du toluène et on ajoute à l'issue de l'étape b) un solvant choisi parmi : du tétrahydrofurane, de l'acétonitrile, de l'acétone, de l'éthanol, de l'eau ou un mélange des ces solvants.

Selon un second mode de réalisation préféré, le milieu réactionnel comprend de l'acétonitrile et on ajoute à l'issue de l'étape b) un solvant choisi parmi : du tétrahydrofurane, du toluène, de l'acétone, de l'éthanol, de l'eau ou un mélange des ces solvants.

L'iodure de 3,7-(dialkylamino)phénothiazine-5-ylium est alors isolé par filtration du milieu réactionnel sur un support de filtration adapté, qui peut être par exemple un verre fritté, une toile filtrante.

La fraction retenue par le filtre est une composition comprenant de l'iodure de 3,7-bis(dialkylamino)-phénothiazine-5-ylium.

Le filtrat est éliminé, tandis que le précipité est lavé une ou plusieurs fois avec un solvant.

De préférence, la filtration est suivie d'une étape de lavage du solide par un solvant qui peut être choisi parmi : du toluène, du tétrahydrofurane, de l'acétonitrile, de l'acétone, de l'éthanol, du dichlorométhane, de l'eau ou un mélange des ces solvants.

L'invention a encore pour objet l'utilisation du procédé décrit ci-dessus pour produire de l'iodure de 3,7-bis(diméthylamino)-phénothiazine-5-ylium d'une bonne pureté chimique, de façon fiable, reproductible et applicable à l'échelle industrielle.

Le procédé de l'invention donne accès à des compositions comprenant de l'iodure de 3,7-bis(diméthylamino)phénothiazine-5-ylium présentant une teneur en iodure de 3,7-bis(diméthylamino)phénothiazine-5-ylium supérieure ou égale à 85 %, préférentiellement supérieure ou égale à 90 %, encore mieux supérieure ou égale à 95 %, et avantageusement supérieure ou égale à 98 %, en % d'aire mesurée en chromatographie liquide haute performance (CHLP) suivant la méthode décrite dans la Pharmacopée européenne 8.6 (publiée en 2015) pour le chlorure de 3,7-bis(diméthylamino)phénothiazine-5-ylium.

Le procédé de l'invention donne accès à des compositions comprenant de l'iodure de 3,7-bis(diméthylamino)phénothiazine-5-ylium présentant une teneur en iodure de 3-diméthylamino, 7-méthylaminophénothiazine-5-ylium inférieure ou égale à 3 %, préférentiellement inférieure ou égale à 2 %, encore mieux inférieure ou égale à 1 %, en % d'aire mesurée en chromatographie liquide haute performance (CHLP) suivant la méthode décrite dans la Pharmacopée européenne 8.6 (publiée en 2015) pour le chlorure de 3,7-bis(diméthylamino)phénothiazine-5-ylium.

Le procédé de l'invention donne ainsi accès à de l'iodure de 3,7-bis(dialkylamino)phénothiazine-5-ylium de pureté élevée et avec des rendements satisfaisants.

Le procédé de l'invention utilise des matières premières et des solvants ne présentant pas de dangerosité particulière. Il fait appel à des étapes facilement extrapolables à plus grande échelle. Par conséquent, ce procédé peut être industrialisé sans difficulté liée à la sécurité, au rendement ou à la qualité du produit.

Ces qualités sont essentielles pour obtenir du chlorure de 3,7-bis(diméthylamino)phénothiazine-5-ylium, également appelé bleu de méthylène de qualité satisfaisante et avec des rendements élevés.

Ce procédé est rapide, fait appel à des matières premières peu couteuses et non toxiques, par conséquent, son application à l'échelle industrielle peut permettre une substitution des procédés actuels de production de bleu de méthylène.

L'invention a en outre pour objet un procédé de fabrication du chlorure de 3,7-bis(diméthylamino)phénothiazine-5-ylium. Ce procédé comprend la production d'iodure de 3,7-bis(diméthylamino)phénothiazine-5-ylium suivant le procédé décrit ci-dessus et une étape supplémentaire de transformation de l'iodure de 3,7-bis(diméthylamino)phénothiazine-5-ylium en chlorure de 3,7-bis(diméthylamino)phénothiazine-5-ylium.

Selon un premier mode de réalisation, la transformation de l'iodure en chlorure de 3,7-bis(diméthylamino)phénothiazine-5-ylium est réalisée par échange d'ions. L'échange de l'ion iodure en ion chlorure est réalisé grâce à une résine échangeuse d'ions comme par exemple une résine Amberlite ®, notamment une résine Amberlite ® IRA958. Une telle étape est bien connue de l'homme du métier, on peut notamment se reporter à Anita Gollmer, et al. Photochem. Photobiol. Sci. ; vol.14, n°2, 1 janvier 2015, p.335-351, pour le détail des conditions opératoires.

Selon un second mode de réalisation, la transformation de l'iodure en chlorure de 3,7-bis(diméthylamino)phénothiazine-5-ylium est réalisée par mise en œuvre du procédé décrit dans la demande WO2008/006979, en particulier dans la partie expérimentale.

De façon brève, l'iodure de 3,7-bis(diméthylamino)phénothiazine-5-ylium est transformé en 3,7-bis(diméthylamino)-10-benzoyl-phénothiazine. Celui-ci est ensuite purifié par filtration sur silice et lavage par du dichlorométhane. Le produit purifié est débenzoylé et oxydé par traitement par une quinone, comme par exemple la 2,3-dichloro-5,6-dicyano-1,4-benzoquinone (DDQ). La 3,7-bis(diméthylamino)phénothiazine est ensuite salifiée par HCl, neutralisée et éventuellement recristallisée.

On obtient ainsi un chlorure de 3,7-bis(diméthylamino)phénothiazine-5-ylium de pureté élevée, en ayant utilisé comme produit de départ une phénothiazine qui est un produit commercial dont la synthèse ne fait pas appel à l'emploi de réactifs hautement toxiques comme les dérivés du chrome qui sont couramment utilisés pour produire du bleu de méthylène de qualité brute.

Par ailleurs, lors de la réaction de benzoylation, la benzoylation de l'iodure de 3,7-bis(diméthylamino)phénothiazine-5-ylium se fait préférentiellement, tandis que l'iodure de 3-diméthylamino, 7-méthylaminophénothiazine-5-ylium, l'iodure de 3,7-bis(méthylamino)phénothiazine-5-ylium, et l'iodure de 3-méthylaminophénothiazine-5-ylium sont très faiblement benzoylés. En outre, lorsqu'ils sont benzoylés, l'iodure de 3-diméthylamino, 7-méthylaminophénothiazine-5-ylium, l'iodure de 3,7-bis(méthylamino)phénothiazine-5-ylium, et l'iodure de 3-méthylaminophénothiazine-5-ylium sont aussi polybenzoylés en proportions non négligeables. Ces caractéristiques spécifiques des contaminants les plus fréquents de l'iodure de 3,7-bis(diméthylamino)phénothiazine-5-ylium facilitent l'élimination de ces contaminants lors de la purification de la forme benzoylée de l'iodure de 3,7-bis(diméthylamino)phénothiazine-5-ylium. Ainsi, le procédé de l'invention, éventuellement suivi par le procédé décrit dans la demande WO2008/006979, donne accès à un bleu de méthylène sensiblement dépourvu de ses habituels contaminants : Azure B, Azure A et Azure C.

Selon un troisième mode de réalisation, la transformation de l'iodure en chlorure de 3,7-bis(diméthylamino)phénothiazine-5-ylium est réalisée par mise en œuvre du procédé décrit dans la demande WO2008/007074, en particulier dans la partie expérimentale.

Ce procédé comprend l'acétylation de l'iodure de 3,7-bis(diméthylamino)phénothiazine-5-ylium en N- 3,7-bis(diméthylamino)-10-acétylphénothiazine. Celui-ci est ensuite purifié par recristallisation dans l'éthanol. Le produit purifié est desacétylé et oxydé par traitement par FeCl₃. Le chlorure de 3,7-bis(diméthylamino)phénothiazinium est éventuellement recristallisé dans de l'eau à pH acide.

Le procédé de l'invention donne accès à des compositions comprenant du chlorure de 3,7-bis(diméthylamino)phénothiazine-5-ylium (Bleu de Méthylène) présentant une teneur en chlorure de 3,7-bis(diméthylamino)phénothiazine-5-ylium supérieure ou égale à 97%, préférentiellement supérieure ou égale à 98%, en % d'aire mesurée en chromatographie liquide haute performance (CHLP) suivant la méthode de la Pharmacopée européenne 8.6 (édition de janvier 2012).

Le procédé de l'invention donne accès à des compositions comprenant du chlorure de 3,7-bis(diméthylamino)phénothiazine-5-ylium présentant une teneur en chlorure de 3-diméthylamino, 7-méthylaminophénothiazine-5-ylium (Azure B) inférieure ou égale à 2%, préférentiellement inférieure ou égale à 1%, en % d'aire mesurée en chromatographie liquide haute performance (CHLP) suivant la méthode de la Pharmacopée européenne 8.6 (publiée en 2015).

Le procédé de l'invention donne également accès à des compositions comprenant du chlorure de 3,7- bis(diméthylamino)phénothiazine-5-ylium comportant peu ou pas d'impuretés métalliques. Le procédé de l'invention donne notamment accès à des compositions comprenant du chlorure de 3,7-bis(diméthylamino)phénothiazine-5-ylium et comportant moins de 200 ppm de contaminants métallique, avantageusement moins de 100 ppm de contaminants métalliques, encore mieux, moins de 50 ppm de contaminants métalliques, et encore plus avantageusement moins de 20 ppm de contaminants métalliques. La teneur en métaux est mesurée suivant la méthode de la Pharmacopée européenne 8.6 (publiée en 2015).

Par contaminants métalliques on entend tous les métaux de la classification périodique des éléments et notamment : Cd, Cr, Hg, Mn, Ni, Sn, Pb, Al, Fe, Cu, Zn, As, Mo, Mg, Ti, V, U, Co. Plus particulièrement, on entend par contaminants métalliques les métaux dits « lourds » et en particulier : Al, Cd, Cr, Cu, Sn, Mn, Hg, Mo, Ni, Pb, Zn.

Le procédé de l'invention permet d'obtenir des compositions comprenant du chlorure de 3,7- bis(diméthylamino)phénothiazine-5-ylium qui sont sensiblement dépourvues d'iodure de 3,7- bis(diméthylamino)phénothiazine-5-ylium.

Le chlorure de 3,7-bis(diméthylamino)phénothiazine-5-ylium, également appelé bleu de méthylène est utilisé depuis des décennies dans le traitement de diverses infections. Il est utilisé comme agent antiseptique, anti infectieux, comme antidote comme traitement de la methémoglobinémie et comme agent de diagnostic.

De façon récente son activité antivirale a été mise en évidence, et il pourrait être utilisé pour la fabrication d'un médicament destiné à être utilisé pour traiter : une tauopathie, une maladie d'agrégation de protéine tau, la maladie de Pick, la paralysie supranucléaire progressive (PSP), la démence fronto-temporale (FTD), la FTD et le parkinsonisme lié au chromosome 17 (FTDP-17), le complexe de désinhibition-démence-parkinsonisme-amyotrophie (DDPAC), la dégénérescence pallido-ponto-nigrale (PPND), le syndrome de Guam-ALS, la dégénérescence pallido-nigro-luysian (PNLD), la dégénérescence cortico-basale (CBD), une déficience cognitive légère (MCI), le cancer de la peau, le mélanome, la méthémoglobinémie, une infection virale, une infection bactérienne, une infection à protozoaires, une infection parasitaire, le paludisme, la leishmaniose viscérale, la maladie africaine du sommeil, la toxoplasmose, la giardiase, la maladie de Chagas, une infection par le virus de l'hépatite C (VHC), une infection par le virus de l'immunodéficience humaine (VIH), une infection par le virus du Nil occidental (WNV), une synucléinopathie, la maladie de Parkinson (PD), la démence à corps de Lewy (DLB), une atrophie multisystématisée (MSA), un parkinsonisme d'origine médicamenteuse, une dysautomie pure (PAF), un choc septique, une réaction hémodynamique excessive, le cancer du sein, les troubles maniaco-dépressifs, la maladie d'Alzheimer (AD) et plus généralement le traitement des maladies dégénératives du système nerveux central.

Le chlorure de 3,7-bis(diméthylamino)phénothiazine-5-ylium pourrait être également utilisé en cosmétique ou pour des produits destinés à une application ophtalmique.

Pour toutes ces applications thérapeutiques, et en particulier dans un contexte de prévention et de traitement de la maladie d'Alzheimer, et plus généralement de traitement des maladies dégénératives du système nerveux central, qui requièrent une administration récurrente de bleu de méthylène pendant des durées prolongées, il est nécessaire de disposer d'un bleu de méthylène ayant un degré de pureté élevé et très peu d'impuretés métalliques.

L'invention a également pour objet un procédé de fabrication d'un médicament, ce procédé comprenant la fabrication de chlorure de 3,7-bis(diméthylamino)phénothiazine-5-ylium et son introduction dans un milieu pharmaceutiquement acceptable.

Ledit médicament peut être sous toute forme adaptée à son utilisation dans ces applications.

En particulier, on peut mentionner : sous forme de comprimé ou de gélule comprenant de 1 à 500 mg de bleu de méthylène ; sous forme de solution aqueuse comprenant du bleu de méthylène à une concentration allant de 0,05% à 2% en g/L.

De telles compositions comprennent, outre le bleu de méthylène, des excipients bien connus de l'homme du métier, tels que par exemple de l'acide citrique et/ou des citrates, un tampon phosphate, des polymères, des dérivés de cellulose, des lipides.

Dans les applications médicales, le bleu de méthylène obtenu par le procédé de l'invention présente l'avantage d'une pureté élevée, ce qui évite d'introduire dans l'organisme des matériaux sans utilité pour l'application.

L'efficacité du procédé de l'invention permet d'accéder à un produit présentant des coûts réduits, facilement reproductible et applicable à l'échelle industrielle.

### Partie expérimentale :

### I- Matériaux et méthodes :

### 1- Matières premières et équipement :

Le diiode a été acheté auprès de la société TCI.

La phénothiazine a été achetée auprès de la société ALFA AESAR, ACROS ORGANICS.

La diméthylamine a été achetée :
- auprès de la société ACROS ORGANICS sous la référence commerciale Diméthylamine 40%wt solution in water
- auprès de la société ACROS ORGANICS sous la référence commerciale Diméthylamine 2M solution in THF
- auprès de la société TCI sous la référence commerciale Dimethylamine, 2M solution in MeOH.

### 2- Méthode d'analyse :

### CLHP/MS

Méthode : Pharmacopée EP 8.6 publiée en 2015
Appareil : HPLC Agilent 1260 + MS Agilent 6120
Colonne : Waters XBridge Phenyl 100x4.6-3.5µm
Détection : 246 nm
Concentration de l'échantillon : 1000 ppm
Solvant de dissolution de l'échantillon : TFA 0.1% aq. /ACN (70/30)
Solvant d'élution : Acétonitrile / Acide trifluoroacétique 0.1% (v/v) dans l'eau
Source d'ionisation pour le MS : Electrospray (ESI)
Analyseur pour le MS : Simple quadripole
Détecteur pour le MS : Multiplicateur d'électrons
Système informatique pour le traitement des données : Agilent Chemstation Open Lab

### II- Protocoles :

### Exemple 1 (Comparatif) : Synthèse de l'iodure de 3,7-bis(diméthylamino)phénothiazine-5-ylium en deux étapes (suivant Anita Gollmer, et al., Photochem. Photobiol. Sci. ; 2014 ; DOI : 10.1039/C4PP00309H ; p.1-47) :

### 1) Synthèse de tétraiodure de phénothiazine-5-ylium (1294-X15)

Dans un tricol de 500 mL, on introduit 10 g de phénothiazine (50 mmol, 1,0 eq.) et 200 mL de dichlorométhane. Le mélange est agité à température ambiante.

Puis, on ajoute sous agitation, et à température ambiante 38,3 g de di-iode (150 mmol, 3,0 eq.).

Le milieu réactionnel est ensuite maintenu sous agitation à température ambiante pendant 2 h.

Le milieu réactionnel est ensuite filtré sur un fritté pore 3.

Le précipité est lavé avec 20 mL de dichlorométhane, puis filtré sur un fritté pore 3.

Le solide est séché à l'étuve à 40°C.

On obtient 31,6 g de tétraiodure de phénothiazine brut, de couleur noire. Le rendement est de 89,5%.

### 2) Transformation du tétraiodure de phénothiazine en iodure de 3,7-bis (diméthylamino)-phénothiazine-5-ylium (1294-Y19)

Dans un tricol de 500 mL, on introduit 4,5 g de périodure de phénothiazine (7,1 mmol, 1,0 eq.) que l'on dissout dans un mélange de méthanol (180 mL) et de dichlorométhane (22,5 mL), sous agitation et à température ambiante.

Sous agitation, 35,5 mL d'une solution de diméthylamine 2N dans du méthanol (71,0 mmol, 10,0 eq.) est coulée en 50 min, conditionné à une température comprise entre 20°C et 25°C.

Le milieu réactionnel est ensuite maintenu sous agitation à température ambiante pendant 6 h à 22°C.

Le milieu réactionnel est ensuite filtré sur un fritté pore 4.

Le produit est obtenu sous forme de poudre et séché dans une étuve ventilée à 40°C.

On obtient 1,39 g d'un solide de couleur noire, le rendement de la réaction est de 47,7%, ce qui correspond à 42,7% par rapport à la quantité totale de phénothiazine engagée dans le procédé.

Le produit est analysé par la méthode CLHP/MS décrite ci-dessus.

La teneur en iodure de 3,7-bis(diméthylamino)phénothiazine-5-ylium dans le solide obtenu est de 85,4%. Parmi les impuretés, on a identifié des dimères de la phénothiazine à hauteur de 11,11%.

### Exemple 2 : Synthèse de l'iodure de 3,7-bis(diméthylamino)phénothiazine-5-ylium en une étape (1294-AD2) :

Dans un tricol de 2 L, on mélange 88 g de di-iode (346,2 mmol, 3,0 eq.) et 1,15 L de toluène. On ajoute, sous agitation, et à température ambiante, 23 g de phénothiazine (115,4 mmol, 1,0 eq.).

Le milieu réactionnel est ensuite maintenu sous agitation à température ambiante pendant 3 h.

On ajoute rapidement (moins d'une minute) dans le milieu réactionnel conditionné à une température de 20 à 25°C, sous agitation, 575 mL d'une solution de diméthylamine 2N dans du méthanol (1154 mmol, 10,0 eq.).

Le milieu réactionnel est ensuite maintenu sous agitation à température ambiante pendant 2 h 30.

Le milieu réactionnel est ensuite filtré sur un fritté pore 3.

Le précipité est lavé une seule fois avec du toluène, puis filtré sur un fritté pore 3.

Le solide est séché au Rotavapor ® à 40°C.

On obtient 40,9 g d'un produit de couleur noire, le rendement obtenu est de 86%.

Le produit est analysé par la méthode CLHP/MS décrite ci-dessus.

La teneur en iodure de 3,7-bis(diméthylamino)phénothiazine-5-ylium est de 85%.

### Exemple 3 : Synthèse de l'iodure de 3,7-bis(diméthylamino)phénothiazine-5-ylium en une étape (1294-AD68) :

Dans un tricol de 250 mL, on introduit 5 g de phénothiazine (25,1 mmol, 1,0 eq.) et 100 mL d'acétonitrile. Le milieu réactionnel est chauffé à 40°C.

On ajoute 19,7 g de di-iode (77,8 mmol, 3,1 eq.) et on poursuit le chauffage pendant 2 h.

Le milieu réactionnel est refroidi à 25°C.

On introduit 31,8 mL de diméthylamine sous forme d'une solution à 40% en masse dans H₂O (250,9 mmol, 10,0 eq.) en maintenant le milieu réactionnel entre 25 et 30°C.

On laisse le milieu pendant 2 h sous agitation à 25°C.

On dilue le milieu avec 100 mL d'acétone et on le laisse 30 min sous agitation à 25°C

Le milieu réactionnel est filtré sur fritté pore 3.

Le précipité est lavé quatre fois avec 20 mL l'acétone.

Le solide est séché une nuit dans une étuve ventilée à 40°C.

On obtient 6,9 g d'un produit de couleur noire, le rendement obtenu est de 67%.

Le produit est analysé par CLHP/MS suivant la méthode décrite ci-dessus, la pureté est de 94,4%.

### Exemple 4 : Synthèse de l'iodure de 3,7-bis(diméthylamino)phénothiazine-5-ylium en une étape (AD97) :

On procède comme à l'exemple 3, avec la différence suivante : On introduit la diméthylamine en maintenant le milieu réactionnel entre 10 et 15°C.

On obtient 4,28 g d'un produit de couleur noire, le rendement obtenu est de 41%.

Le produit est analysé par CLHP/MS suivant la méthode décrite ci-dessus, la pureté est de 98,3%.

### Exemple 5 : Synthèse de l'iodure de 3,7-bis(diméthylamino)phénothiazine-5-ylium en une étape (AD98) :

On procède comme à l'exemple 3, avec la différence suivante : On introduit la diméthylamine en maintenant le milieu réactionnel entre 35 et 40°C.

On obtient 6,21 g d'un produit de couleur noire, le rendement obtenu est de 60%.

Le produit est analysé par CLHP/MS suivant la méthode décrite ci-dessus, la pureté est de 88,1%.

### Exemple 6 : Synthèse de l'iodure de 3,7-bis(diméthylamino)phénothiazine-5-ylium en une étape (AD99) :

On procède comme à l'exemple 3, avec la différence suivante : On introduit la diméthylamine en maintenant le milieu réactionnel entre 60 et 70°C.

On obtient 4,82 g d'un produit de couleur noire, le rendement obtenu est de 47%.

Le produit est analysé par CLHP/MS suivant la méthode décrite ci-dessus, la pureté est de 73,3%.

### Exemple 7 : Synthèse de l'iodure de 3,7-bis(diméthylamino)phénothiazine-5-ylium en une étape (AD55)

Dans un tricol de 500 mL, on introduit 5 g de phénothiazine (25,1 mmol, 1,0 eq.) et 100 mL d'acétonitrile. Le milieu réactionnel est chauffé à 40°C.

On ajoute 19,1 g de di-iode (75,3 mmol, 3,0 eq.) et on poursuit le chauffage pendant 2 h.

Le milieu réactionnel est refroidi à 18°C.

On introduit 31,8 mL de diméthylamine sous forme d'une solution à 40% en masse dans H₂O (250,9 mmol, 10,0 eq.) en 5 min. La réaction est exothermique et on observe à la fin de l'ajout une température du milieu réactionnel de 24°C.

On laisse le milieu pendant 2 h sous agitation à température ambiante.

On dilue le milieu avec 100 mL d'acétone et on le laisse 30 min sous agitation à température ambiante.

Le précipité est filtré sur fritté pore 3 et lavé avec de l'acétone.

Le solide est séché au Rotavapor ® à 40°C.

On obtient 6,2 g d'un produit de couleur noire, le rendement obtenu est de 60%.

Le produit est analysé par CLHP/MS suivant la méthode décrite ci-dessus, la pureté est de 96,2%.

### Exemple 8 : Synthèse de l'iodure de 3,7-bis(diméthylamino)phénothiazine-5-ylium en une étape (AD62)

Dans un tricol de 500 mL, on introduit 5 g de phénothiazine (25,1 mmol, 1,0 eq.) et 100 mL d'acétonitrile. Le milieu réactionnel est chauffé à 40°C.

On ajoute 25,5 g de di-iode (100,4 mmol, 4,0 eq.) et on poursuit le chauffage pendant 2 h.

Le milieu réactionnel est refroidi à 20-25°C.

On introduit 31,8 mL de diméthylamine sous forme d'une solution à 40% en masse dans H₂O (250,9 mmol, 10,0 eq.) en 5 minutes. La réaction est exothermique et on observe à la fin de l'ajout une température du milieu réactionnel T<30°C.

On laisse le milieu pendant 2 h sous agitation à température ambiante.

On dilue le milieu avec 100 mL d'acétone et on le laisse 30 min sous agitation à température ambiante.

Le précipité est filtré sur fritté pore 3 et lavé avec de l'acétone.

Le solide est séché au Rotavapor ® à 40°C.

On obtient 4,0 g d'un produit de couleur noire, le rendement obtenu est de 39%.

Le produit est analysé par CLHP/MS suivant la méthode décrite ci-dessus, la pureté est de 98,1%.

### Exemple 9 : Synthèse du 3,7-bis(diméthylamino)-10-benzoyl-phénothiazine (1294-AD86)

Dans un réacteur de 6 litres, on introduit 100 g de phénothiazine (502 mmol, 1,0 eq.), 394,8 g de di-iode (1555 mmol, 3,1 eq.) et 2 L d'acétonitrile.

Le milieu réactionnel est chauffé à 40°C pendant 2 h.

Le milieu réactionnel est refroidi à 25°C.

On introduit 633 mL de diméthylamine sous forme d'une solution à 40% en masse dans H₂O (5020 mmol, 10,0 eq.) en maintenant le milieu réactionnel entre 25 et 30°C.

On laisse le milieu pendant 2 h sous agitation à 25°C.

On dilue le milieu avec 2 L d'acétone et on le laisse 30 min sous agitation à 25°C

Le précipité obtenu est filtré sur fritté pore 3 puis lavé 4 fois avec 400 mL d'acétone.

Le solide est séché une nuit dans une étuve ventilée à 40°C.

On obtient 128,4 g d'un produit de couleur noire, le rendement obtenu est de 62,2%.

Le produit est analysé par CLHP/MS suivant la méthode décrite ci-dessus, la pureté est de 95.5%.

### Exemple 10 : Synthèse du 3,7-bis(diméthylamino)-10-benzoyl-phénothiazine (1294-Z18)

Dans un tricol de 2 L, on introduit 30 g de l'iodure de 3,7-bis(diméthylamino)phénothiazine-5-ylium (72,9 mmol, 1,0 eq.) préparé à l'exemple 9 dans 600 mL d'eau. Le mélange est agité sous flux de N₂ jusqu'à dissolution complète, puis il est refroidi à 12°C. On ajoute ensuite 44,8 g d'hydrosulfite de sodium 85% (218,7 mmol, 3,0 eq.) dans la solution en 5 min et sous agitation.

Le milieu réactionnel est agité à 12°C pendant 30 min. On ajoute alors 58,32 g d'une solution aqueuse de soude à 30% (437,4 mmol, 6,0 eq.) en 10 min. 50,8 mL de chlorure de benzoyle (437,4 mmol, 6,0 eq.) sont ajoutés dans le milieu réactionnel en 25 min.

Le milieu réactionnel est alors agité pendant 2h à 12°C. On ajoute 600 mL de dichlorométhane, le milieu réactionnel est agité pendant quelques minutes, puis on ajoute 150 mL d'une solution aqueuse de soude à 30% et on laisse sous agitation pendant 1h à 12°C.

Le milieu réactionnel est extrait dans une ampoule à décanter de 2 L sous N₂. La phase aqueuse est extraite deux fois avec 300 mL de dichlorométhane. La phase organique est lavée deux fois avec 150 mL de solution aqueuse de soude 1N, passée sur silice puis l'évaporée sous vide. Le solide est solubilisé dans 300 mL d'éthanol, on place la solution pendant 45 min à -20°C. Le précipité est filtré sur fritté pore 3, lavé à l'éthanol gelé puis séché une nuit dans une étuve ventilée à 40°C.

Le dosage de l'iode est réalisé par ICP/MS. Le produit obtenu contient 1203 ppm d'iode résiduel.

Les impuretés métalliques sont analysées par spectrométrie de masse à plasma à couplage inductif, équipée d'une cellule de collision (CCT mode). L'indium est utilisé comme étalon interne. L'or est utilisé comme stabilisant du mercure. Une minéralisation des échantillons est réalisée sous haute pression avec un four à micro-ondes de laboratoire. La méthode utilisée est une méthodologie en ajout dosé.

Les résultats sont reportés dans le tableau ci-dessous :

| Paramètre | Résultat |
|---|---|
| Ruthénium | < 0,03 ppm |
| Rhodium | < 0,03 ppm |
| Cadmium | < 0,03 ppm |
| Iridium | < 0,03 ppm |
| Mercure | < 0,03 ppm |
| Chrome | 1,0 ppm |
| Manganèse | < 0,15 ppm |
| Nickel | < 0,15 ppm |
| Etain | < 0,15 ppm |
| Plomb | < 0,15 ppm |
| Aluminium | <1,2 ppm |
| Fer | <1,2 ppm |
| Cuivre | <0,6 |
| Zinc | <0,6 |
| Arsenic | <0,15 |
| Molybdène | <0,15 |

### Exemple 11 : Synthèse du chlorure de 3,7-bis(diméthylamino)phénothiazine-5-ylium (1294-AG10)

### Débenzoylation :

Dans un tricol de 500 mL, on introduit 5 g de 3,7-bis(diméthylamino)-10-benzoyl-phénothiazine (12,8 mmol, 1,0 eq.) obtenu à l'exemple 10 dans 200 mL d'acétonitrile. Le milieu réactionnel est refroidi à -20°C. On prépare une solution de 2,97g de 2,3-dichloro-5,6-dicyano-1,4-benzoquinone (DDQ) (13,1 mmol, 1,02 eq.) dans 13,4 mL d'acétonitrile et on la conditionne à -20°C.

La solution froide de DDQ est ensuite versée dans le tricol et on laisse sous agitation pendant 3h à -20°C. On ajoute 50 mL d'acétate d'éthyle (AE) et on laisse sous agitation pendant 30 min à -20°C. Le milieu réactionnel est filtré sur fritté pore 3 puis lavé deux fois avec 10 mL d'acétate d'éthyle. Le précipité obtenu est lavé avec 50 mL d'un mélange AE/THF (25/75), et essoré.

### Salification :

Le solide obtenu est repris dans 40 mL d'AE. Le mélange est refroidi à -20°C et on ajoute rapidement 39 mL d'acétate d'éthyle/HCl (4.3M) (165,12 mmol, 12,9 eq.). Le milieu réactionnel est laissé sous agitation pendant 3 h à -20°C. Le précipité obtenu est filtré sur fritté pore 3. Le solide obtenu est repris dans 75 mL d'acétate d'éthyle. Le mélange est agité 30 min à -20°C puis filtré sur fritté pore 3.

### Neutralisation :

On effectue une mesure du pH sur 100 mg de précipité dans 20 mL d'eau puis le pH est ajusté à 3.8 avec 200 µL de NaOH 0,2 M. Le produit obtenu est repris dans 50 mL d'acétone puis refroidi à - 15°C. On ajoute 2,3 mL de NaOH 2M, volume précédemment déterminé, puis on agite 2 h à -15°C. Le précipité obtenu est filtré sur fritté pore 3. Le solide est repris dans 20 mL d'acétone. Le milieu est agité 30 min à - 15°C, filtré sur fritté pore 3 puis on effectue une mesure du pH dans les mêmes conditions que précédemment. Le précipité est séché une nuit dans une étuve ventilée à 40°C.

### Purification et hydratation :

Dans un tricol de 100 mL, on introduit 2,67 g de bleu de méthylène salifié dans 43 mL d'un mélange DCM/EtOH (50/50). Le milieu est chauffé à 43°C sous agitation puis filtré à chaud sur fritté pore 3. 1,33 mL d'eau sont ajoutés dans le filtrat puis le dichlorométhane est évaporé sous vide. 75 mL d'acétate d'éthyle sont ajoutés dans le milieu refroidi à -20°C puis laissé sous agitation une nuit. Le précipité obtenu est filtré sur fritté pore 3 puis réempaté dans 40 mL d'une solution THF/AE (75/25). On obtient après filtration et séchage, deux jours dans une étuve à 40°C, 1,45 g de bleu de méthylène.

Le dosage des iodures est réalisé par chromatographie ionique. Le produit obtenu contient moins de 0.015 ppm d'iodure résiduel.

## Revendications

1. Procédé de préparation d'iodure de 3,7-bis(diméthylamino)phénothiazine-5-ylium, ce procédé utilisant la phénothiazine comme produit de départ et comprenant les étapes suivantes :
a) Le traitement de la phénothiazine par du di-iode,
b) Le traitement du milieu réactionnel directement issu de l'étape a) par de la diméthylamine.

2. Procédé selon la revendication 1, dans lequel le traitement par le di-iode est mis en œuvre avec une quantité de di-iode par rapport à la phénothiazine allant de 2,5 équivalents molaires à 3,5 équivalents molaires.

3. Procédé selon l'une quelconque des revendications précédentes, dans lequel avant l'étape b), le milieu réactionnel issu de l'étape a) est conditionné à une température allant de 5°C à 50°C, de préférence de 10°C à 45°C, encore mieux de 20°C à 35°C.

4. Procédé selon l'une quelconque des revendications précédentes, dans lequel le traitement par de la diméthylamine est mis en œuvre avec au moins 7 équivalents molaires de diméthylamine par rapport à la phénothiazine.

5. Procédé selon l'une quelconque des revendications précédentes, dans lequel à l'étape a), le solvant est choisi parmi : un solvant aromatique ou l'acétonitrile, ou leurs mélanges, de préférence le toluène ou l'acétonitrile, ou leurs mélanges.

6. Procédé selon l'une quelconque des revendications précédentes, dans lequel à l'étape b), la diméthylamine est introduite dans le milieu réactionnel sous forme de solution dans de l'eau.

7. Procédé selon l'une quelconque des revendications précédentes, dans lequel il se forme un précipité à l'issue du traitement de l'étape b), ledit précipité étant récupéré par filtration.

8. Procédé selon l'une quelconque des revendications précédentes, dans lequel la phénothiazine utilisée comme produit de départ présente une pureté organique supérieure ou égale à 98% mesurée par chromatographie liquide à haute performance, avec une détection à 246 nm.

9. Procédé selon l'une quelconque des revendications précédentes, dans lequel la phénothiazine utilisée comme produit de départ comporte moins de 20 ppm de métaux.

10. Utilisation du procédé selon l'une quelconque des revendications précédentes pour produire une composition comprenant de l'iodure de 3,7-bis(dialkylamino)phénothiazine-5-ylium dans laquelle l'iodure de 3,7-bis(dialkylamino)phénothiazine-5-ylium représente au moins 95% de la composition, le % étant mesuré par CLHP avec une détection à 246 nm.

11. Procédé de fabrication du chlorure de 3,7-bis(diméthylamino)phénothiazine-5-ylium, ce procédé comprenant :
i) La production d'iodure de 3,7-bis(diméthylamino)phénothiazine-5-ylium suivant l'une quelconque des revendications 1 à 9,
ii) La transformation de l'iodure de 3,7-bis(diméthylamino)phénothiazine-5-ylium en chlorure de 3,7-bis(diméthylamino)phénothiazine-5-ylium.

12. Procédé selon la revendication 11, pour produire une composition présentant une teneur en chlorure de 3,7-bis(diméthylamino)phénothiazine-5-ylium supérieure ou égale à 97%, en % d'aire mesurée en chromatographie liquide haute performance suivant la méthode de la Pharmacopée européenne 8.6.

13. Procédé selon la revendication 11 ou selon la revendication 12 pour produire une composition comprenant du chlorure de 3,7-bis(diméthylamino)phénothiazine-5-ylium et comportant moins de 20 ppm de métaux.

14. Procédé de fabrication d'un médicament comprenant du chlorure de 3,7-bis(diméthylamino)phénothiazine-5-ylium, ce procédé comprenant la production du chlorure de 3,7-bis(diméthylamino)phénothiazine-5-ylium suivant l'une quelconque des revendications 11 à 13, et son introduction dans un milieu pharmaceutiquement acceptable.

15. Procédé selon la revendication 14, pour la fabrication d'un médicament destiné à la prévention ou au traitement d'une pathologie sélectionnée parmi: une tauopathie, une maladie d'agrégation de protéine tau, la maladie de Pick, la paralysie supranucléaire progressive (PSP), la démence fronto-temporale (FTD), la FTD et le parkinsonisme lié au chromosome 17 (FTDP-17), le complexe de désinhibition-démence-parkinsonisme-amyotrophie (DDPAC), la dégénérescence pallido-ponto-nigrale (PPND), le syndrome de Guam-ALS, la dégénérescence pallido-nigro-luysian (PNLD), la dégénérescence cortico-basale (CBD), une déficience cognitive légère (MCI), le cancer de la peau, le mélanome, la méthémoglobinémie, une infection virale, une infection bactérienne, une infection à protozoaires, une infection parasitaire, le paludisme, la leishmaniose viscérale, la maladie africaine du sommeil, la toxoplasmose, la giardiase, la maladie de Chagas, une infection par le virus de l'hépatite C (VHC), une infection par le virus de l'immunodéficience humaine (VIH), une infection par le virus du Nil occidental (WNV), une synucléinopathie, la maladie de Parkinson (PD), la démence à corps de Lewy (DLB), une atrophie multisystématisée (MSA), une parkinsonisme d'origine médicamenteuse, une dysautomie pure (PAF), un choc septique, une réaction hémodynamique excessive, le cancer du sein, les troubles maniaco-dépressifs, la maladie d'Alzheimer (AD) et plus généralement le traitement des maladies dégénératives du système nerveux central.

## Patentansprüche

1. Verfahren zur Herstellung von 3,7-Bis(dimethylamino)phenothiazin-5-yliumiodid, wobei das Verfahren Phenothiazin als Ausgangsprodukt verwendet und die folgenden Schritte umfasst:
a) die Behandlung von Phenothiazin mit Diiod,
b) die Behandlung des Reaktionsmediums direkt nach Schritt a) mit Dimethylamin.

2. Verfahren nach Anspruch 1, wobei die Behandlung mit Diiod mit einer Menge an Diiod in Bezug auf Phenothiazin von 2,5 Moläquivalenten bis 3,5 Moläquivalenten durchgeführt wird.

3. Verfahren nach einem der vorhergehenden Ansprüche, wobei vor Schritt b) das Reaktionsmedium aus Schritt a) bei einer Temperatur von 5°C bis 50°C, vorzugsweise von 10°C bis 45°C, noch besser von 20°C bis 35°C, konditioniert wird.

4. Verfahren nach einem der vorhergehenden Ansprüche, wobei die Behandlung mit Dimethylamin mit mindestens 7 Moläquivalenten Dimethylamin in Bezug auf Phenothiazin durchgeführt wird.

5. Verfahren nach einem der vorhergehenden Ansprüche, wobei in Schritt a) das Lösungsmittel aus den folgenden ausgewählt ist: einem aromatischen Lösungsmittel oder Acetonitril oder deren Gemischen, vorzugsweise Toluol oder Acetonitril oder deren Gemischen.

6. Verfahren nach einem der vorhergehenden Ansprüche, wobei in Schritt a) Dimethylamin in das Reaktionsmedium in Form einer Lösung in Wasser eingebracht wird.

7. Verfahren nach einem der vorhergehenden Ansprüche, wobei sich am Ende der Behandlung von Schritt b) ein Niederschlag bildet, wobei der Niederschlag durch Filtration gewonnen wird.

8. Verfahren nach einem der vorhergehenden Ansprüche, wobei das als Ausgangsprodukt verwendete Phenothiazin eine durch Hochleistungsflüssigchromatographie mit Nachweis bei 246 nm gemessene organische Reinheit von mehr als oder gleich 98% aufweist.

9. Verfahren nach einem der vorhergehenden Ansprüche, wobei das als Ausgangsprodukt verwendete Phenothiazin weniger als 20 ppm Metalle enthält.

10. Verwendung des Verfahrens nach einem der vorhergehenden Ansprüche zur Herstellung einer Zusammensetzung, die 3,7-Bis(dialkylamino)phenothiazin-5-yliumiodid umfasst, wobei das 3,7-Bis(dialkylamino)phenothiazin-5-yliumiodid mindestens 95% der Zusammensetzung ausmacht, wobei die % durch HPLC mit Nachweis bei 246 nm gemessen werden.

11. Verfahren zur Herstellung von 3,7-Bis(dimethylamino)phenothiazin-5-yliumchlorid, wobei das Verfahren Folgendes umfasst:
i) die Herstellung von 3,7-Bis(dimethylamino)phenothiazin-5-yliumiodid nach einem der Ansprüche 1 bis 9,
ii) die Umwandlung von 3,7-Bis(dimethylamino)phenothiazin-5-yliumiodid in 3,7-Bis(dimethylamino)phenothiazin-5-yliumchlorid.

12. Verfahren nach Anspruch 11 zur Herstellung einer Zusammensetzung, die einen Gehalt an 3,7-Bis(dimethylamino)phenothiazin-5-yliumchlorid von mehr als oder gleich 97 % in % der mittels Hochleistungsflüssigchromatographie nach dem Verfahren des Europäischen Arzneibuchs 8.6 gemessenen Fläche aufweist.

13. Verfahren nach Anspruch 11 oder nach Anspruch 12 zur Herstellung einer Zusammensetzung, die 3,7-Bis(dimethylamino)phenothiazin-5-yliumchlorid und weniger als 20 ppm Metalle umfasst.

14. Verfahren zur Herstellung eines Medikaments, das 3,7-Bis(dimethylamino)phenothiazin-5-yliumchlorid umfasst, wobei das Verfahren die Herstellung von 3,7-Bis(dimethylamino)phenothiazin-5-yliumchlorid nach einem der Ansprüche 11 bis 13 sowie dessen Einbringen in ein pharmazeutisch akzeptables Medium umfasst.

15. Verfahren nach Anspruch 14 zur Herstellung eines Medikaments zur Vorbeugung oder Behandlung einer Pathologie, ausgewählt aus: einer Tauopathie, einer Tau-Protein-Aggregationskrankheit, Morbus Pick, progressiver supranukleärer Paralyse (PSP), frontotemporaler Demenz (FTD), FTD und Parkinson in Zusammenhang mit Chromosom 17 (FTDP-17), dem Enthemmung(Disinhibition)-Demenz-Parkinson-Amyotrophie-Komplex (DDPAC), pallidopontonigraler Degeneration (PPND), Guam-ALS-Syndrom, Pallido-Nigro-Luys-Degeneration (PNLD), kortiko-basaler Degeneration (CBD), einer leichten kognitiven Beeinträchtigung (MCI), Hautkrebs, Melanom, Methämoglobinämie, einer Virusinfektion, einer bakteriellen Infektion, einer Protozoeninfektion, einer Parasiteninfektion, Malaria, viszeraler Leishmaniose, afrikanischer Schlafkrankheit, Toxoplasmose, Giardiasis, Chagas-Krankheit, einer Infektion mit dem Hepatitis-C-Virus (HCV), einer Infektion mit dem humanen Immundefizienzvirus (HIV), einer Infektion mit dem West-Nil-Virus (WNV), einer Synukleinopathie, Parkinson-Krankheit (PD), Lewy-Körper-Demenz (LBD), einer Multisystematrophie (MSA), durch Medikamente hervorgerufenem Parkinsonismus, reinem autonomem Versagen (PAF), septischem Schock, übermäßiger hämodynamischer Reaktion, Brustkrebs, manisch-depressiven Störungen, Alzheimer-Krankheit (AD), und allgemeiner die Behandlung degenerativer Erkrankungen des zentralen Nervensystems.

## Claims

1. A process for preparing 3,7-bis(dimethylamino)phenothiazin-5-ylium iodide, this process using phenothiazine as starting product and comprising the following steps:
a) treating phenothiazine with diiodine,
b) treating the reaction medium directly resulting from step a) with dimethylamine.

2. The process as claimed in claim 1, wherein the treatment with diiodine is carried out with an amount of diiodine, relative to the phenothiazine, ranging from 2.5 molar equivalents to 3.5 molar equivalents.

3. The process as claimed in either one of the preceding claims, wherein, before step b), the reaction medium resulting from step a) is conditioned at a temperature ranging from 5°C to 50°C, preferably from 10°C to 45°C, even better still from 20°C to 35°C.

4. The process as claimed in any one of the preceding claims, wherein the treatment with dimethylamine is carried out with at least 7 molar equivalents of dimethylamine relative to the phenothiazine.

5. The process as claimed in any one of the preceding claims, wherein, in step a), the solvent is chosen from: an aromatic solvent or acetonitrile, or mixtures thereof, preferably toluene or acetonitrile, or mixtures thereof.

6. The process as claimed in any one of the preceding claims, wherein, in step b), the dimethylamine is introduced into the reaction medium in the form of a solution in water.

7. The process as claimed in any one of the preceding claims, wherein a precipitate forms at the outcome of the treatment of step b), said precipitate being recovered by filtration.

8. The process as claimed in any one of the preceding claims, wherein the phenothiazine used as starting product has an organic purity greater than or equal to 98% measured by high performance liquid chromatography, with detection at 246 nm.

9. The process as claimed in any one of the preceding claims, wherein the phenothiazine used as starting product comprises less than 20 ppm of metals.

10. The use of the process as claimed in any one of the preceding claims, for producing a composition comprising 3,7-bis(dialkylamino)phenothiazin-5-ylium iodide, wherein the 3,7-bis(dialkylamino)phenothiazin-5-ylium iodide represents at least 95% of the composition, the % being measured by HPLC with detection at 246 nm.

11. A process for producing 3,7-bis(dimethylamino)phenothiazin-5-ylium chloride, this process comprising:
a) producing 3,7-bis(dimethylamino)phenothiazin-5-ylium iodide as claimed in any one of claims 1 to 9,
b) converting the 3,7-bis(dimethylamino)phenothiazin-5-ylium iodide into 3,7-bis(dimethylamino)phenothiazin-5-ylium chloride.

12. The process as claimed in claim 11, for producing a composition having a 3,7-bis(dimethylamino)phenothiazin-5-ylium chloride content of greater than or equal to 97%, as % of area measured by high performance liquid chromatography according to the method of the European Pharmacopeia 8.6.

13. The process as claimed in claim 11 or claim 12, for producing a composition comprising 3,7-bis(dimethylamino)phenothiazin-5-ylium chloride and comprising less than 20 ppm of metals.

14. A process for producing a medicament comprising 3,7-bis(dimethylamino)phenothiazin-5-ylium chloride, this process comprising the production of 3,7-bis(dimethylamino)phenothiazin-5-ylium chloride as claimed in claims 11 to 13, and the introduction thereof into a pharmaceutically acceptable medium.

15. The process as claimed in claim 14, for producing a medicament intended for the prevention or treatment of a pathological condition selected from: a tauopathy, a tau protein aggregation disease, Pick's disease, progressive supranuclear palsy (PSP), frontotemporal dementia (FTD), FTD and parkinsonism linked to chromosome 17 (FTDP-17), disinhibition-dementia-parkinsonism-amyotrophy complex (DDPAC), pallido-ponto-nigral degeneration (PPND), guam-ALS syndrome, pallido-nigro-luysian degeneration (PNLD), corticobasal degeneration (CBD), mild cognitive impairment (MCI), skin cancer, melanoma, methemoglobinemia, a viral infection, a bacterial infection, a protozoan infection, a parasite infection, malaria, visceral leishmaniosis, African sleeping sickness, toxoplasmosis, giardiasis, Chagas disease, a hepatitis C virus (HCV) infection, a human immunodeficiency virus (HIV) infection, a West Nile virus (WNV) infection, synucleinopathy, Parkinson's disease (PD), Lewy body dementia (DLB), multiple system atrophy (MSA), drug-induced parkinsonism, pure autonomic failure (PAF), septic shock, excessive hemodynamic reaction, breast cancer, manic-depressive disorders, Alzheimer's disease (AD) and more generally the treatment of degenerative diseases of the central nervous system.
